# EUROPEAN PATENT SPECIFICATION

(11) **EP 0 785 787 B1**
(45) Date of publication and mention of the grant of the patent: **12.12.2001**
(21) Application number: 95935280.8
(22) Date of filing: 13.10.1995
(51) Int. Cl.: A61K 31/55

(54) **USE OF 1,5-BENZODIAZEPINE DERIVATIVES FOR THE CONTROL OF GASTRIC EMPTYING IN PATIENTS WITH NON-INSULIN DEPENDENT DIABETES MELLITUS**
VERWENDUNG VON 1,5-BENZODIAZEPINDERIVATEN ZUR KONTROLLE DES MAGEN ENTLEERENS BEI NICHT INSULINABHÄNGIGEM DIABETES MELLITUS
UTILISATION DE DERIVES DE BENZODIAZEPINE-1, 5 POUR LA REGULATION DE VIDANGE GASTRIQUE CHEZ LES PATIENTS PRESENTANT DES CONDITIONS DE DIABETE NON INSULINO-DEPENDANT

(30) Priority: 14.10.1994 GB 9420747
(43) Date of publication of application: 30.07.1997
(73) Proprietor: GLAXO WELLCOME INC., Research Triangle Park, North Carolina 27709 (US)
(72) Inventor: HANLEY, Rochelle, Research Triangle Park, NC 27709 (US)
(74) Representative: Learoyd, Stephanie Anne
(86) International application number: US9512830
(87) International publication number: WO9611691

(56) References cited:
- WO-A-94/24149
- US-A- 5 187 154
- SCAND J GASTROENTEROL, MAY 1993, 28 (5) P401-7, NORWAY, KONTUREK JW ET AL 'Cholecystokinin in the regulation of gastric acid and endocrine pancreatic secretion in humans.'
- J PHYSIOL PARIS, 1993, 87 (5) P291-300, FRANCE, SCARPIGNATO C ET AL 'Effect of CCK and its antagonists on gastric emptying.'

## Description

This invention relates to a new medical use of 1,5-benzodiazepine derivatives which exhibit CCK-A agonist activity. More particularly it relates to the use of such compounds in the manufacture of a therapeutic agent to control gastric emptying in patients having an early non-insulin-dependent diabetic condition.

Cholecystokinins (CCK) and gastrin are structurally related peptides which exist in gastrointestinal tissue and in the central nervous system. Cholecystokinins include CCK-33, a neuropeptide of thirty-three amino acids in its originally isolated form, its carboxyl terminal octapeptide, CCK-8 (also a naturally occurring neuropeptide), and 39- and 12-amino acid forms. Gastrin occurs in 34-, 17- and 14- amino acid forms, with the minimum active sequence being the C-terminal tetrapeptide, Trp-Met-Asp-Phe-NH₂ (CCK-4) which is the common structural element shared by both CCK and gastrin.

CCK and gastrin are gastrointestinal hormones and neurotransmitters in the neural and peripheral systems and perform their respective biological roles by binding to particular receptors located at various sites throughout the body. There are at least two subtypes of cholecystokinin receptors termed CCK-A and CCK-B and both are found in the periphery and in the central nervous system.

The CCK-A receptor, commonly referred to as the "peripheral-type" receptor, is primarily found in the pancreas, gallbladder, ileum, pyloric sphincter and on vagal afferent nerve fibers. Type-A CCK receptors are also found in the brain in discrete regions and serve to provide a number of CNS effects. Due to the ability of CCK-8 and Type-A CCK-selective agonists to suppress food intake in several animal species, considerable interest has been generated toward the development of new substances which function as Type-A receptor-selective CCK agonists in order to serve as anorectic agents.

The CCK-B or gastrin receptors are found in peripheral neurons, gastrointestinal smooth muscle and gastrointestinal mucosa, most notably in parietal cells, ECL cells, D cells and chief cells. CCK-B receptors also predominate in the brain and have been implicated in the regulation of anxiety, arousal and the action of neuroleptic agents.

U.S. Patent No. 4,988,692, to Gasc, et al. describes a group of 3-acylamino 1-alkyl-5-phenyl 1,5-benzodiazepine derivatives which behave as cholecystokinin antagonists to reverse or block the effects of the endogenous hormone at its receptors.

US Patent No. 4,490,304 and PTC applications No's WO90/06937 and WO91/19733 describe peptide derivatives that exhibit CCK-A agonist activity. Such compounds have been disclosed for appetite regulation as well as the treatment and/or prevention of gastrointestinal disorders or disorders of the central nervous in animals and, more particularly, humans.

US Patent No. 5,187,154 describes the use of the neuropeptide cholecystokinin (CCK) to control gastric emptying in patients having an early non-insulin-dependent diabetic condition and exhibiting rapid gastric emptying. Further the specification teaches that compounds which inhibit gastric emptying may be useful to alleviate or eliminate symptoms associated with early or pre-diabetes. Particular symptoms include elevated blood glucose and insulin levels, insulin resistance, increased susceptibility to infection or glycosuria while also maintaining gastric emptying within normal levels.

We have now discovered that a novel group of 3-amino 1,5-benzodiazepine compounds which exhibit agonist activity for the CCK-A receptor delay or inhibit gastric emptying and thus may be used to treat patents having non-insulin-dependent diabetic conditions and exhibiting rapid gastric emptying.

The present invention thus provides for the use of compounds of the general Formula (I) 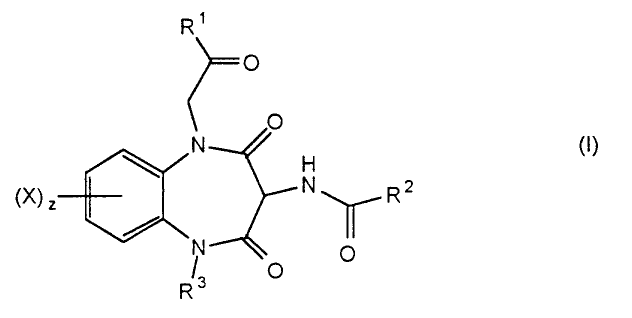 and physiologically salts and solvate thereof wherein:
X is either hydrogen, trifluoromethyl, alkyl, C₁₋₄alkylthio, -O(C₁₋₄alkyl) or halogen;
R¹ is either Formula II or -NR⁴R⁵; 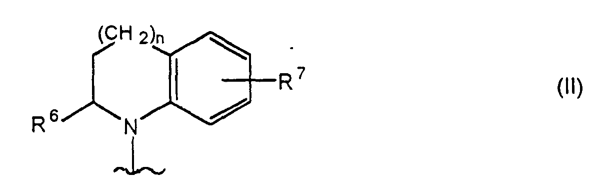
R² is either:
   (1) a heterocycle linked at its 2- position and selected from pyrrole, tetrahydropyrrole, indole, benzofuran, thiophene, benzothiophene, indoline, quinoline or 4-oxobenzopyran and wherein said pyrrole, tetrahydropyrrole, indole or indoline may optionally be substituted on the ring nitrogen thereof by the group R⁸ as defined hereunder and said indole, indoline, quinoline, benzofuran, benzothiophene or 4-oxo-benzopyran may optionally be substituted in the benzo ring thereof by the group R⁹ as defined hereunder or
   (2) phenyl or phenyl mono- or disubstituted independently with halogen, hydroxy, cyano, carboxy, -O(C₁₋₄alkyl), -O(CH₂C₆H₅), -COO(C₁₋₄alkyl), amino, dimethylamino, -NHR¹⁰, 1-pyrrolidinyl or tetrazolyl; or
   (3) pyridine or pyridinyl mono- or disubstituted independently with halogen, methyl, hydroxy, nitro, cyano, carboxy, -O(C₁₋₄ alkyl), -O(CH₂C₆H₅), -COO(C₁₋₄alkyl), amino or dimethylamino; or
   (4) -NHR¹¹ where R¹¹ is defined hereinunder or R¹¹ is 7-indazolyl containing a group R¹⁰ at the N-1 position;
R³ is hydrogen, C₁₋₆alkyl, C₃₋₆cycloalkyl, phenyl or phenyl mono- or disubstituted independently with halogen;
R⁴ is independently C₃₋₆alkyl, C₃₋₆cycloalkyl, C₃₋₆alkenyl, phenyl, -(CH₂)ₚCN or -(CH₂)ₚCOO(C₁₋₄alkyl) and R⁵ is independently C₃₋₆alkyl, C₃₋₆cycloalkyl, C₃₋₆ alkenyl, benzyl, phenyl or phenyl mono- or disubstituted independently with C₁₋₃alkyl, optionally substituted by 1 or more fluorine atoms cyano, hydroxy, dimethylamino, -O(C₁₋₄alkyl), -O(CH₂C₆H₅), -NH(C₁₋₄alkyl), -COO(C₁₋₄alkyl),-N(C₁₋₄alkyl)₂ pyrrolidino, morpholino or halogen or R⁴ is C₁₋₂alkyl and R⁵ is phenyl substituted at the 2- or 4- position with chloro, methyl, methoxy or methoxycarbonyl;
R⁶ is hydrogen or methyl;
R⁷ is hydrogen, hydroxy, fluoro, dimethylamino, -O(C₁₋₄alkyl) or -O(CH₂C₆H₅);
R⁸ is -(CH₂)_{b}COOH;
R⁹ is methyl, chloro, nitro, hydroxy, methoxy or -NHR¹⁰;
R¹⁰ is hydrogen, acetyl, C₁₋₄alkyl, -SO₃H, -SO₂CH₃, -SO₂CF₃ or -SO₂C₆H₅, C₁₋₄alkoxycarbonyl;
R¹¹ is phenyl or phenyl mono- or disubstituted independently with fluorine, trifluoromethoxy, C₁₋₄alkylthio, -(CH₂)_{c}COOH, -(CH₂)_{c}COO(C₁₋₄alkyl), -(CH₂)_{c}SCH₃, -(CH₂)_{c}SOCH₃, -(CH₂)_{c}SO₂CH₃, -(CH₂)_{c}CONH₂, -SCH₂COOH, -CONH(SO₂CH₃), -CONH(SO₂CF₃), -(CH₂)_{c}N(C₁₋₄alkyl)₂,-(CH₂)_{c}NH(SO₂CF₃), -(CH₂)_{c}N(SO₂CF₃)(C₁₋₄alkyl), -(CH₂)_{c}SO₂NHCO(C₁₋₄alkyl), -(CH₂)_{c}SO₂N (C₁₋₄alkyl)CO(C₁₋₄alkyl), -(CH₂)_{c}CONHSO₂(C₁₋₄alkyl), -(CH₂)_{c}CON (C₁₋₄alkyl)SO₂(C₁₋₄alkyl), -(CH₂)_{c}OR¹² -(CH₂)_{c}NHR¹⁰ or phenyl monosubstituted with -(CH₂)_{c}(tetrazolyl), -(CH₂)_{c} (carboxamidotetrazolyl) or -(CH₂)_{c}(pyrrolidinyl) or R¹¹ is selected from pyridine or pyridinyl mono- or disubstituted independently with halogen, methyl, hydroxy, nitro, cyano, carboxy, -O(C₁₋₄ alkyl), amino, dimethylamino, -NHR¹⁰;
R¹² is hydrogen, C₁₋₆alkyl, C₃₋₆cycloalkyl, -CH₂C₆H₅, -CH₂COOH, -CH₂CONH₂, -CH₂CONH(C₁₋₄alkyl), -CH₂CON(C₁₋₄alkyl)₂ or 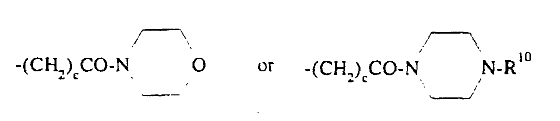
z is 1 or 2;
n is 1 or 2;
p is an integer from 1-4;
b is an integer from 0-3; and
c is 0 or 1,
in the manufacture of a therapeutic agent for controlling gastric emptying in patients having an early non-insulin-dependent diabetic condition and exhibiting rapid gastric emptying.

When R¹ represents the group of Formula (II), examples of such a group include those wherein R⁶ is hydrogen or more particularly methyl, R⁷ is hydrogen, hydroxyl, methoxy, or fluorine, and n is 1.

When R¹ represents the group NR⁴R⁵ , examples of suitable groups include those wherein R⁴ represent C₃₋₆ alkyl, such as propyl or isopropyl, cyclohexyl or phenyl and R⁵ represents C₃₋₆ alkyl, benzyl or phenyl optionally substituted in the para- position by hydroxy, dimethylamino methoxy, trifluoromethyl, fluorine, pyrrolidino or morpholino. Within this group, particularly useful R¹ groups include those wherein R⁴ is propyl and, more particularly, isopropyl and R⁵ represents phenyl or phenyl substituted in the para-position by groups selected from hydroxy, methoxy dimethylamino, fluorine, or morpholino.

Examples of particularly suitable R¹ groups include those wherein R¹ is the group of Formula (II) wherein R₆ is methyl, n is 1 and R⁷ is hydrogen, hydroxy, fluorine or methoxy or R¹ is the group NR⁴R⁵ wherein R⁴ is propyl or isopropyl and R⁵ is phenyl optionally substituted in the para position by a group selected from hydroxy, methoxy, fluoro, trifluoromethyl, dimethylamino, pyrrolidino or morpholino.

When R² represents a group selected from indole, indoline, benzofuran, benzothiophene, quinoline or 4-oxobenzopyran, the optional substituent R⁹ is conveniently a group selected from hydrogen, methyl, methoxy, hydroxy, nitro or amino and, where appropriate, the optional substituent on nitrogen, (R⁸ ), is-CH₂CO₂H.

When R² is an optionally substituted phenyl group, this is conveniently phenyl or phenyl substituted by one or two groups, which may be the same or different and selected from chlorine, fluorine, amino, hydroxy or carboxyl.

When R² represents the group NHR¹¹, R¹¹ is conveniently phenyl (optionally substituted by fluoro, hydroxy, amino, dimethylamino, trifluoromethylsulphonylamino, C₁₋₄ alkoxycarbonyl, carboxy, 1H-tetrazol-5-yl, acetylamino or OR¹² wherein R¹² represents hydrogen, methyl, benzyl, CH₂CO₂H, CH₂CONH₂, CH₂CONHCH₃, CH₂CON(CH₃)₂ 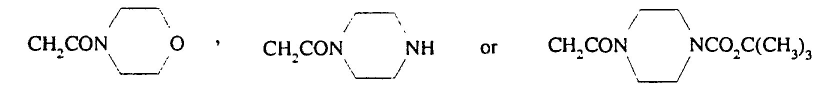 ) or a 7-indazolyl group wherein the N-1 substituent, (R¹⁰), is hydrogen.

When R¹¹ is a mono substituted phenyl group, the substituted is conveniently in the meta- position.

Examples of particularly suitable R² groups includes indole, benzofuran, thiophene, benzothiophene, indoline, quinoline, 4-oxobenzopyran, an optionally substituted phenyl group or the group NHR¹¹. Conveniently, R² is selected from the group indole, indoline or benzofuran, an optionally substituted phenyl group or the group NHR¹¹. More particularly, R² represents an indole, an optionally substituted phenyl or NHR¹¹.

When R₃ represents C₁₋₆ alkyl, examples of suitable groups include methyl, ethyl, propyl, isopropyl, butyl, t-butyl or isoamyl.

When R₃ represents C₃₋₆ cycloalkyl, examples of suitable groups include cyclopropyl, cyclopentyl or cyclohexyl.

When R₃ represents phenyl, mono or disubstituted by independently with halogen, examples of suitable groups include those wherein the halogen substituent is fluorine e.g., 2-fluorophenyl or 4 fluorophenyl.

Examples of particularly suitable R³ groups include hydrogen, methyl, cyclohexyl, 2-fluorophenyl or phenyl, and more particularly, phenyl.

A particularly useful group of compounds for use according to the invention include those wherein R¹ represents the group of Formula (II) wherein R⁶ is methyl, n is 1 and R⁷ is hydrogen, fluorine, hydroxy or methoxy, or more particularly NR⁴R⁵ wherein R⁴ is propyl or isopropyl and R⁵ is phenyl optionally substituted in the para position by a group selected from hydroxy, methoxy, fluoro, dimethylamino or morpholino; R² represents phenyl (optionally substituted independently by one or two groups selected from chlorine, fluorine, hydroxy, amine or carboxy), NHR¹¹ wherein R¹¹ represents phenyl (optionally substituted by amino, dimethylamino, trifluoromethyl- sulphonylamino, carboxy, 1H-tetrazol-5-yl, acetylamino or OR¹² wherein R¹² represents hydrogen, methyl, benzyl, CH₂CO₂H, CH₂CONH₂, CH₂CONHCH₃, CH₂CON(CH₃)₂, 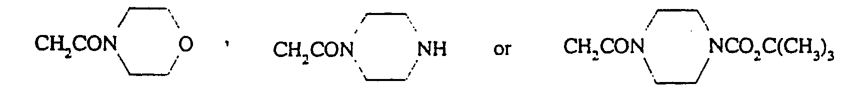 and
wherein the substituent is preferably in the meta- position) or an indole wherein the nitrogen atom is optionally subtituted by the group -CH₂CO₂H and the benzo ring is optionally substituted by chlorine, methyl, methoxy, nitro, hydroxy or amino; R³ represents hydrogen, methyl, cyclohexyl, 2- fluorophenyl or phenyl or, more particularly, 2 fluorophenyl or phenyl; and X represents fluorine and z is 1 or, more particularly, X is hydrogen;

A particularly interesting class of compounds for use in the present invention are those wherein R² is an indole group. A preferred group of compounds within this class are those wherein the indole group is substituted on the nitrogen atom by the group -CH₂CO₂H or, more preferably, the nitrogen atom is unsubstituted, and benzo ring of the indole group is optionally substituted by a group selected from chlorine, methyl, methoxy, nitro, hydroxy or amino.

A particularly preferred compound for use in the invention which is hereinafter referred to as compound 'A' is:
1H-Indole-2-carboxylic acid {1-[Isopropyl-(4-methoxyphenyl)carbamoyl-methyl]-2,4-dioxo-5-phenyl-2,3,4,5-tetrahydro-1H-benzo[b][1,4]diazepin-3-yl}-amide and enantiomers thereof.

As provided herein, the term alkyl is generally intended to mean both straight chain and branched chain aliphatic isomers of the corresponding alkyl. For example, C₁₋₆alkyl is intended to include methyl, ethyl, n-propyl, isopropyl, n-butyl, isobutyl, tertbutyl, n-pentyl, etc.

The term cycloalkyl, as provided herein, is intended to mean all alicyclic isomers of the corresponding alkyl. For example, the term C₃₋₆ alkyl, as provided herein, is intended to include such groups as cyclopropyl, cyclopentyl and cyclohexyl.

The term halogen is intended to mean F, Cl, Br or I.

The term tetrazole as a group or part of a group refers to the (1 H)-tetrazol-5-yl grouping and tautomers thereof.

Those skilled in the art will recognize that stereocenters exist in compounds of Formula (I). Accordingly, the present invention includes all possible stereoisomers and geometric isomers of Formula (I) and includes not only racemic compounds but also the optically active isomers as well. When a compound of Formula (I) is desired as a single enantiomer, it may be obtained either by resolution of the final product or by stereospecific synthesis from either isomerically pure starting material or any convenient intermediate. Resolution of the final product, an intermediate or a starting material may be effected by any suitable method known in the art. See, for example, Stereochemistry of Carbon Compounds by E. L. Eliel (Mcgraw Hill, 1962) and Tables of Resolving Agents by S. H. Wilen. Additionally, in situations where tautomers of the compounds of Formula (I) are possible, the present invention is intended to include all tautomeric forms of the compounds.

It will also be appreciated by those skilled in the art that the compounds of the present invention may also be utilized in the form of a pharmaceutically acceptable salt or solvate thereof. The physiologically acceptable salts of the compounds of Formula (I) include conventional salts formed from pharmaceutically acceptable inorganic or organic acids as well as quaternary ammonium acid addition salts. More specific examples of suitable salts include hydrochloric, hydrobromic, sulphuric, phosphoric, nitric, perchloric, fumaric, acetic, propionic, succinic, glycolic, formic, lactic, maleic, tartaric, citric, pamoic, malonic, hydroxymaleic, phenylacetic, glutamic, benzoic, salicylic, fumaric, toluenesulphonic, methanesulphonic, naphthalene-2-sulphonic, benzenesulphonic and the like. Other acids such as oxalic, while not in themselves pharmaceutically acceptable, may be useful in the preparation of salts useful as intermediates in obtaining the compounds of the invention and their pharmaceutically acceptable salts. References hereinafter to a compound according to the invention include both compounds of Formula (I) and their pharmaceutically acceptable salts and solvates.

CCK-A agonist activity of the compounds of formula (I) may be determined by standard procedures.

The relative affinities of compounds of formula (I) for the CCK-A and CCK-B receptors may also be determined using known conventional procedures such as described by Fornos et al J. Pharmacol Exp. Ther., 1992 261, 1056-1063.

The compounds of formula (I) inhibit or delay gastric emptying and thus may be used to alleviate or eliminate symptoms associated with early or prediabetes, particularly for non-insulin dependent diabetes. Such symptoms include elevated blood glucose and insulin levels, insulin resistance, increased susceptibility to infection and/or glycosuria while also maintaining gastric emptying with normal levels.

The ability of compounds of formula (I) to inhibit or delay gastric emptying may be determined using standard tests. Thus for example rats deprived for food for 18hr were pretreated with the test compound administered i.p at a pre-set time (20 mins) before being given a methyl cellulose meal which was administered by the gavage route. The meal contains a marker element such as Phenol Red. After specific predetermined time intervals the rats are sacrificed and the amount of the meal in the stomach is determined by measuring the concentration of the marker substance present. This value is then compared with a control animal which was not pre-treated with the test compound. In this test the preferred compound of formula (I) compound 'A' when administered i.p at doses of 1µmole/kg 20 min before gavage of test meal (1.5% methyl cellulose). completely inhibited gastric emptying 30mins after administration of the test meal. Lower doses of the compound 'A' 0.01 and 0.1 µmoles per kg i.p also resulted in a significant reduction in gastric emptying.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02 - 5000 mg per day, e.g., 1-1500 mg per day. The desired dose may conveniently be presented in a single dose or as divided doses administered at appropriate intervals, for example as two, three, four or more sub-doses per day.

While it is possible that compounds of formula (I) may be therapeutically administered as the raw chemical, it is preferable to present the active ingredient as a pharmaceutical formulation. Accordingly, the present invention further provides for the use in the present invention of a compound of Formula (I) or a pharmaceutically acceptable salt thereof together with one or more pharmaceutically acceptable carriers therefore and, optionally, other therapeutic and/or prophylactic ingredients for the manufacture of a therapeutic agent. The carrier(s) must be "acceptable" in the sense of being compatible with the other ingredients of the formulation and not deleterious to the recipient thereof.

Formulations used as a therapeutic agent according to the present invention include those especially formulated for oral, buccal, parenteral, implant, or rectal administration, however, oral administration is preferred. For buccal administration, the composition may take the form of tablets or lozenges formulated in conventional manner. Tablets and capsules for oral administration may contain conventional excipients such as binding agents, (for example, syrup, acacia, gelatin, sorbitol, tragacanth, mucilage of starch or polyvinylpyrrolidone), fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulphate. The tablets may be coated according to methods well-known in the art. Such tablet coatings conveniently include conventional enteric coatings known to those skilled in the art e.g. cellulose acetate phthalate, polyvinyl acetate phthalate, shellac, styrene maleic acid copolymers, methyacrylic acid copolymers and hydroxypropyl methyl cellulose phthalate.

Alternatively, the compounds of the present invention may be incorporated into oral liquid preparations such as aqueous or oily suspensions, solutions, emulsions, syrups or elixirs, for example. Moreover, formulations containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides.

For oral administration the compounds of formula (I) are preferably formulated as enteric coated tablets or enteric capsules.

### Pharmacy

**Tablet**

| | |
|---|---|
| Active Ingredient | 50 mg |
| Lactose anhydrous USP | 163 mg |
| Microcrystalline Cellulose NF | 69 mg |
| Pregelatinized starch Ph. Eur. | 15 mg |
| Magnesium stearate USP | 3 mg |
| Compression weight | 300 mg |

The active ingredient, microcrystalline cellulose, lactose and pregletinized starch are sieved through a 500 micron sieve and blended in a suitable mixer. The magnesium starate is sieved through a 250 micron sieve and blended with the active blend. The blend is compressed into tablets using suitable punches, then coated in a conventional manner with an enteric coating such as cellulose acetate phthalate.

## Claims

1. The use of a compound of Formula (1) 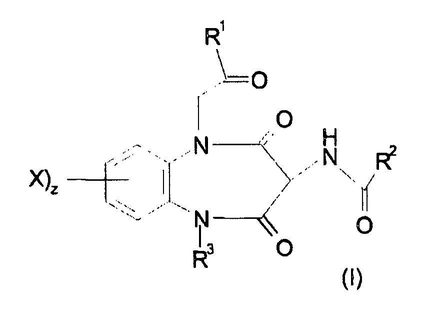 and physiologically salts and solvate thereof wherein:
X is either hydrogen, trifluoromethyl, alkyl, C₁₋₄alkylthio, -O(C₁₋₄alkyl) or halogen;
R¹ is either Formula II or -NR⁴R⁵·; 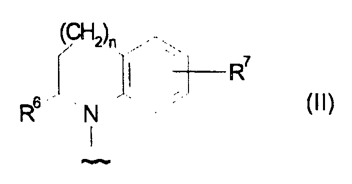
R² is either:
(1) a heterocycle linked at its 2- position and selected from pyrrole, tetrahydropyrrole, indole, benzofuran, thiophene, benzothiophene, indoline, quinoline or 4-oxobenzopyran and wherein said pyrrole, tetrahydropyrrole, indole or indoline may optionally be substituted on the ring nitrogen thereof by the group R⁸ as defined hereunder and said indole, indoline, quinoline, benzofuran, benzothiophene or 4-oxobenzopyran may optionally be substituted in the benzo ring thereof by the group R⁹ as defined hereunder or
(2) phenyl or phenyl mono- or disubstituted independently with halogen, hydroxy, cyano, carboxy, -O(C₁₋₄alkyl), -O(CH₂C₆H₅), -COO(C₁₋₄alkyl), amino, dimethylamino, -NHR¹⁰, 1-pyrrolidinyl or tetrazolyl; or
(3) pyridine or pyridinyl mono- or disubstituted independently with halogen, methyl, hydroxy, nitro, cyano, carboxy, -O(C₁₋₄ alkyl), -O(CH₂C₆H₅),-COO(C₁₋₄alkyl), amino or dimethylamino; or
(4) -NHR¹¹ where R¹¹ is defined hereinunder or R¹¹ is 7-indazolyl containing a group R¹⁰ at the N-1 position;
R³ is hydrogen, C₁₋₆alkyl, C₃₋₆cycloalkyl, phenyl or phenyl mono- or disubstituted independently with halogen;
R⁴ is independently C₃₋₆alkyl, C₃₋₆cycloalkyl, C₃₋₆alkenyl, phenyl, -(CH₂)ₚCN or -(CH₂)ₚCOO(C₁₋₄alkyl) and R⁵ is independently C₃₋₆alkyl, C₃₋₆cycloalkyl, C₃₋₆ alkenyl, benzyl, phenyl or phenyl mono- or disubstituted independently with C₁₋₃alkyl, cyano, hydroxy, dimethylamino, -O(C₁₋₄alkyl), -O(CH₂C₆H₅),-NH(C₁₋₄alkyl), -COO(C₁₋₄alkyl), -N(C₁₋₄alkyl)₂ pyrrolidino, morpholino or halogen or R⁴ is C₁₋₂alkyl and R⁵ is phenyl substituted at the 2- or 4- position with chloro, methyl, methoxy or methoxycarbonyl;
R⁶ is hydrogen or methyl;
R⁷ is hydrogen, hydroxy, fluoro, dimethylamino, -O(C₁₋₄alkyl) or -O(CH₂C₆H₅);
R⁸ is -(CH₂)_{b}COOH;
R⁹ is methyl, chloro, nitro, hydroxy, methoxy or -NHR¹⁰;
R¹⁰ is hydrogen, acetyl, C₁₋₄alkyl, -SO₃H, -SO₂CH₃, -SO₂CF₃ or -SO₂C₆H₅, C₁₋₄alkoxycarbonyl;
R¹¹ is phenyl or phenyl mono- or disubstituted independently with fluorine, trifluoromethoxy, C₁₋₄alkylthio, -(CH₂)_{c}COOH, -(CH₂)_{c}COO(C₁₋₄alkyl), -(CH₂)_{c}SCH₃, -(CH₂)_{c}SOCH₃, -(CH₂)_{c}SO₂CH₃, -(CH₂)_{c}CONH₂, -SCH₂COOH, -CONH(SO₂CH₃), -CONH(SO₂CF₃), -(CH₂)_{c}N(C₁₋₄alkyl)₂, -(CH₂)_{c}NH(SO₂CF₃),-(CH₂)_{c}N(SO₂CF₃)(C₁₋₄alkyl), -(CH₂)_{c}SO₂NHCO(C₁₋₄alkyl), -(CH₂)_{c}SO₂N(C₁₋₄alkyl)CO(C₁₋₄alkyl), -(CH₂)_{c}CONHSO₂(C₁₋₄alkyl), -(CH₂)_{c}CON(C₁₋₄alkyl)SO₂(C₁₋₄alkyl), -(CH₂)_{c}OR¹²-(CH₂)_{c}NHR¹⁰ or phenyl monosubstituted with -(CH₂)_{c}(tetrazolyl), -(CH₂)_{c} (carboxamidotetrazolyl) or-(CH₂)_{c}(pyrrolidinyl) or R¹¹ is selected from pyridine or pyridinyl mono- or disubstituted independently with halogen, methyl, hydroxy, nitro, cyano, carboxy, -O(C₁₋₄ alkyl), amino, dimethylamino, -NHR¹⁰;
R¹² is hydrogen, C₁₋₆alkyl, C₃₋₆cycloalkyl, -CH₂C₆H₅, -CH₂COOH,-CH₂CONH₂, -CH₂CONH(C₁₋₄alkyl), -CH₂CON(C₁₋₄alkyl)₂ or 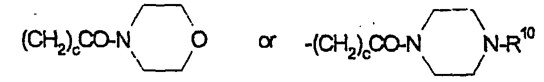
z is 1 or 2;
n is 1 or 2;
p is an integer from 1-4;
b is an integer from 0-3; and
c is 0 or 1.
in the manufacture of a therapeutic agent for controlling gastric emptying in patients having an early non-insulin-dependent diabetic condition and exhibiting rapid gastric emptying.

2. The use of a compound as claimed in Claim 1 wherein R¹ represents the group of Formula (II) wherein R⁶ is methyl, R⁷ is hydrogen, hydroxyl, methoxy or fluorine and n is 1 or R¹ represents the group NR⁴R⁵ wherein R⁴ represents C₃₋₆ alkyl, cyclohexyl or phenyl, and R⁵ represents C₃₋₆ alkyl or phenyl optionally substituted in the para position by hydroxy, dimethylamino, methoxy, fluorine, pyrrolidino or morpholino.

3. The use of a compound as claimed in Claims 1 or 2 wherein R¹ represents the group NR⁴R⁵ and R⁴ represents propyl or isopropyl and R⁵ represents phenyl or phenyl substituted in the para position by a group selected from hydroxy, methoxy, dimethylamino, fluorine, or morpholino.

4. The use of a compound as claimed in any of Claims 1 to 3 wherein R² represents a group selected from phenyl (optionally substituted by one or two groups which may be the same or different and selected from chlorine, fluorine, amino, hydroxy or carboxy,) or NHR¹¹ wherein R¹¹ is phenyl (optionally substituted by fluoro, hydroxy, amino, dimethylamino, trifluoromethylsulphonylamino, C₁₋₄ alkoxycarbonyl, carboxy, 1H-tetrazol-5-yl, acetylamino or OR¹² wherein R¹² represents hydrogen, methyl, benzyl, CH₂CO₂H, CH₂CONH₂, CH₂CONHCH₃, CH₂CON(CH₃)₂ 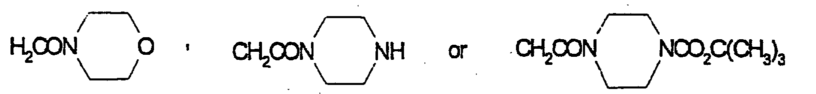 or 7-indazolyl wherein the N-1 substituent R¹⁰ is hydrogen, or R² represents an indole group wherein the nitrogen atom is optionally substituted by the group-CH₂CO₂H and the benzo ring is optionally substituted by a group selected from chlorine, methyl, methoxy, nitro, hydroxy or amino.

5. The use of a compound as claimed in any of Claims 1-4 wherein R² represents an indole group which is unsubstituted on the nitrogen atom and in which the benzo ring thereof is optionally substituted by a group selected from chlorine, methyl, methoxy, nitro, hydroxy or amino.

6. The use of a compound as claimed in any of Claims 1-5 wherein R³ represents hydrogen, methyl, cyclohexyl,2-fluorophenyl or phenyl.

7. The use of a compound as claimed in any of Claims 1-6 wherein R³ represents phenyl.

8. The use of a compound as claimed in any of Claims 1-7 wherein X represents hydrogen.

9. The use of a compound as claimed in Claim 1 wherein R¹ represents NR⁴R⁵ and R⁴ represents isopropyl and R⁵ represents p-methoxyphenyl; R² represents an unsubstituted 2-indole group; R³ represents phenyl and X represents hydrogen and enantiomers thereof.

## Patentansprüche

1. Verwendung einer Verbindung der Formel (I): 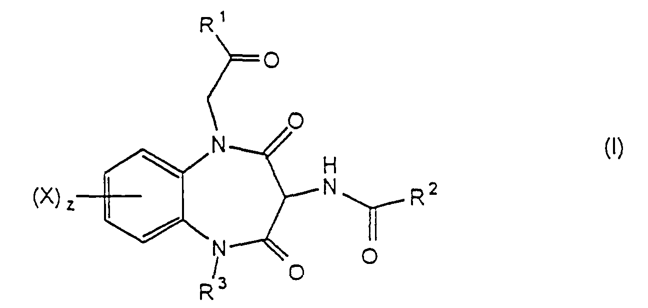 und physiologischer Salze und Solvate davon, worin:
X entweder Wasserstoff, Trifluormethyl, Alkyl, C₁₋₄-Alkylthio, -O(C₁₋₄-Alkyl) oder Halogen ist;
R¹ entweder Formel (II) oder -NR⁴R⁵ ist; 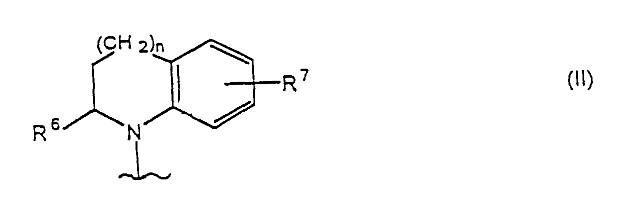
R² entweder:
(1) ein in seiner 2-Position gebundener Heterocyclus und aus Pyrrol, Tetrahydropyrrol, Indol, Benzofuran, Thiophen, Benzothiophen, Indolin, Chinolin oder 4-Oxobenzopyran ausgewählt ist und worin das Pyrrol, Tetrahydropyrrol, Indol oder Indolin gegebenenfalls am Ring-Stickstoff davon mit der Gruppe R⁸ wie nachfolgend definiert substituiert sein kann und das Indol, Indolin, Chinolin, Benzofuran, Benzothiophen oder 4-Oxo-benzopyran gegebenenfalls im Benzoring davon mit der Gruppe R⁹ wie nachfolgend definiert substituiert sein kann, oder
(2) Phenyl oder unabhängig mit Halogen, Hydroxy, Cyano, Carboxy, -O(C₁₋₄-Alkyl), -(CH₂C₆H₅), -COO(C₁₋₄-Alkyl), Amino, Dimethylamino, -NHR¹⁰, 1-Pyrrolidinyl oder Tetrazoyl mono- oder disubstituiertes Phenyl ist; oder
(3) Pyridin oder unabhängig mit Halogen, Methyl, Hydroxy, Nitro, Cyano, Carboxy, -O(C₁₋₄-Alkyl), -O(CH₂C₆H₅), -COO(C₁₋₄-Alkyl), Amino oder Dimethylamino mono- oder disubstituiertes Pyridinyl ist; oder
(4) -NHR¹¹ ist, worin R¹¹ nachfolgend definiert ist oder R¹¹ 7-Indazolyl ist, das eine Gruppe R¹⁰ an der N-1-Position enthält;
R³ Wasserstoff, C₁₋₆-Alkyl, C₃₋₆-Cycloalkyl, Phenyl oder unabhängig mit Halogen mono- oder disubstituiertes Phenyl ist;
R⁴ unabhängig C₃₋₆-Alkyl, C₃₋₆-Cycloalkyl, C₃₋₆-Alkenyl, Phenyl, -(CH₂)ₚCN oder -(CH₂)ₚCOO(C₁₋₄-Alkyl) ist und R⁵ unabhängig C₃₋₆-Alkyl, C₃₋₆-Cycloalkyl, C₃₋₆-Alkenyl, Benzyl, Phenyl oder unabhängig mit C₁₋₃-Alkyl, Cyano, Hydroxy, Dimethylamino, -O(C₁₋₄-Alkyl), -O(CH₂C₆H₅), -NH(C₁₋₄-Alkyl), -COO(C₁₋₄-Alkyl), -N(C₁₋₄-Alkyl)₂, Pyrrolidino, Morpholino oder Halogen mono- oder disubstituiertes Phenyl ist, oder R⁴ C₁₋₂-Alkyl ist und
R⁵ in der 2- oder 4-Position mit Chlor, Methyl, Methoxy oder Methoxycarbonyl substituiertes Phenyl ist;
R⁶ Wasserstoff oder Methyl ist;
R⁷ Wasserstoff, Hydroxy, Fluor, Dimethylamino, -O(C₁₋₄-Alkyl) oder -O(CH₂C₆H₅) ist;
R⁸ -(CH₂)_{b}COOH ist;
R⁹ Methyl, Chlor, Nitro, Hydroxy, Methoxy oder -NHR¹⁰ ist;
R¹⁰ Wasserstoff, Acetyl, C₁₋₄-Alkyl, -SO₃H, -SO₂CH₃, -SO₂CF₃ oder -SO₂C₆H₅, C₁₋₄-Alkoxycarbonyl ist;
R¹¹ Phenyl oder unabhängig mit Fluor, Trifluormethoxy, C₁₋₄-Alkylthio, -(CH₂)_{c}COOH, -(CH₂)_{c}COO(C₁₋₄-Alkyl), -(CH₂)_{c}SCH₃, -(CH₂)_{c}SOCH₃, -(CH₂)_{c}SO₂CH₃, -(CH₂)_{c}CONH₂, -SCH₂COOH, -CONH(SO₂CH₃), -CONH(SO₂CF₃), -(CH₂)_{c}N(C₁₋₄-Alkyl)₂, -(CH₂)_{c}NH(SO₂CF₃), -(CH₂)_{c}N(SO₂CF₃)(C₁₋₄-Alkyl), -(CH₂)_{c}SO₂NHCO(C₁₋₄-Alkyl), -(CH₂)_{c}SO₂N(C₁₋₄-Alkyl)CO(C₁₋₄-alkyl), -(CH₂)_{c}CONHSO₂(C₁₋₄-Alkyl), -(CH₂)_{c}CON(C₁₋₄-Alkyl)SO₂(C₁₋₄-alkyl), -(CH₂)_{c}OR¹² oder -(CH₂)_{c}NHR¹⁰ mono- oder disubstituiertes Phenyl oder mit -(CH₂)_{c}(Tetrazolyl), -(CH₂)_{c}(Carboxamidotetrazolyl) oder-(CH₂)_{c}(Pyrrolidinyl) monosubstituiertes Phenyl ist, oder R¹¹ aus Pyridin oder unabhängig mit Halogen, Methyl, Hydroxy, Nitro, Cyano, Carboxy, -O(C₁₋₄-Alkyl), Amino, Dimethylamino oder -NHR¹⁰ mono- oder disubstituiertem Pyridinyl ausgewählt ist;
R¹² Wasserstoff, C₁₋₆-Alkyl, C₃₋₆-Cycloalkyl, -CH₂C₆H₅, -CH₂COOH, -CH₂CONH₂, -CH₂CONH(C₁₋₄-Alkyl), -CH₂CON(C₁₋₄-Alkyl)₂ oder 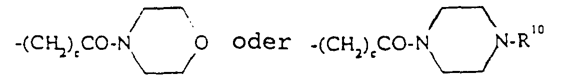 ist;
z 1 oder 2 ist;
n 1 oder 2 ist;
p eine ganze Zahl von 1 bis 4 ist;
b eine ganze Zahl von 0 bis 3 ist; und
c 0 oder 1 ist,
in der Herstellung eines Therapeutikums zur Regulierung der Magenentleerung bei Patienten mit einem frühen nicht-insulinabhängigen diabetischen Zustand, die eine schnelle Magenentleerung aufweisen.

2. Verwendung einer Verbindung gemäß Anspruch 1, worin R¹ die Gruppe der Formel (II) dargestellt, worin R⁶ Methyl ist, R⁷ Wasserstoff, Hydroxyl, Methoxy oder Fluor ist und n 1 ist, oder R¹ die Gruppe NR⁴R⁵ darstellt, worin R⁴ C₃₋₆-Alkyl, Cyclohexyl oder Phenyl darstellt und R⁵ C₃₋₆-Alkyl oder gegebenenfalls in der para-Position mit Hydroxy, Dimethylamino, Methoxy, Fluor, Pyrrolidino oder Morpholino substituiertes Phenyl darstellt.

3. Verwendung einer Verbindung gemäß Anspruch 1 oder 2, worin R¹ die Gruppe NR⁴R⁵ darstellt und R⁴ Propyl oder Isopropyl darstellt und R⁵ Phenyl oder in der para-Position mit einer aus Hydroxy, Methoxy, Dimethylamino, Fluor oder Morpholino ausgewählten Gruppe substituiertes Phenyl darstellt.

4. Verwendung einer Verbindung gemäß einem der Ansprüche 1 bis 3, worin R² eine Gruppe darstellt, die aus Phenyl (gegebenenfalls substituiert mit einer oder zwei Gruppen, die gleich oder verschieden sein können und aus Chlor, Fluor, Amino, Hydroxy oder Carboxy ausgewählt sind) oder NHR¹¹ ausgewählt ist, worin R¹¹ Phenyl (gegebenenfalls substituiert mit Fluor, Hydroxy, Amino, Dimethylamino, Trifluormethylsulfonylamino, C₁₋₄-Alkoxycarbonyl, Carboxy, 1H-Tetrazol-5-yl, Acetylamino oder OR¹², worin R¹² Wasserstoff, Methyl, Benzyl, CH₂CO₂H, CH₂CONH₂, CH₂CONHCH₃, CH₂CON(CH₃)₂, 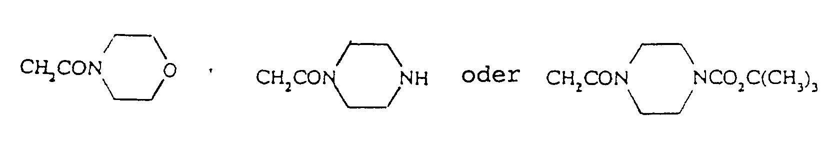 darstellt) oder 7-Indazolyl ist, worin der N-1-Substituent R¹⁰ Wasserstoff ist, oder R² eine Indol-Gruppe darstellt, worin das Stickstoffatom gegebenenfalls mit der Gruppe -CH₂CO₂H substituiert ist und der Benzoring gegebenenfalls mit einer aus Chlor, Methyl, Methoxy, Nitro, Hydroxy oder Amino ausgewählten Gruppe substituiert ist.

5. Verwendung einer Verbindung gemäß einem der Ansprüche 1 bis 4, worin R² eine Indol-Gruppe darstellt, die am Stickstoffatom unsubstituiert ist und in der der Benzoring davon gegebenenfalls mit einer aus Chlor, Methyl, Methoxy, Nitro, Hydroxy oder Amino ausgewählten Gruppe substituiert ist.

6. Verwendung einer Verbindung gemäß einem der Ansprüche 1 bis 5, worin R³ Wasserstoff, Methyl, Cyclohexyl, 2-Fluorphenyl oder Phenyl darstellt.

7. Verwendung einer Verbindung gemäß einem der Ansprüche 1 bis 6, worin R³ Phenyl darstellt.

8. Verwendung einer Verbindung gemäß einem der Ansprüche 1 bis 7, worin X Wasserstoff darstellt.

9. Verwendung einer Verbindung gemäß Anspruch 1, worin R¹ NR⁴R⁵ darstellt und R⁴ Isopropyl darstellt und R⁵ p-Methoxyphenyl darstellt; R² eine unsubstituierte 2-Indol-Gruppe darstellt; R³ Phenyl darstellt und X Wasserstoff darstellt, und der Enantiomere davon.

## Revendications

1. Utilisation d'un composé répondant à la formule (I) 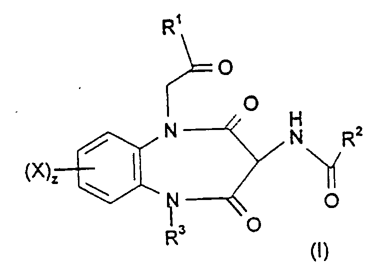 ainsi que de ses sels et de ses solvates physiologiquement acceptables, dans laquelle
X représente un atome d'hydrogène, un groupe trifluorométhyle, un groupe alkyle, un groupe alkyl(en C₁-C₄)thio, un groupe O-alkyle en C₁-C₄ ou un atome d'halogène;
R¹ soit répond à la formule II, soit représente un groupe -NR⁴R⁵; 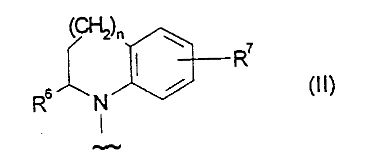
R² représente, soit
(1) un groupe hétérocyclique lié en position 2 et choisi parmi le groupe comprenant un groupe pyrrole, un groupe tétrahydropyrrole, un groupe indole, un groupe benzofuranne, un groupe thiophène, un groupe benzothiophène, un groupe indoline, un groupe quinoléine ou un groupe 4-oxobenzopyrane, et dans lequel ledit groupe pyrrole, ledit groupe tétrahydropyrrole, ledit groupe indole ou ledit groupe indoline peut, le cas échéant, être substitué sur son atome d'azote nucléaire par le groupe R⁸ tel que défini ci-dessous, et ledit groupe indole, ledit groupe indoline, ledit groupe quinoléine, ledit groupe benzofuranne, ledit groupe benzothiophène ou ledit groupe 4-oxobenzopyrane peut, le cas échéant, être substitué dans son noyau benzénique par le groupe R⁹ tel que défini ci-dessous, soit
(2) un groupe phényle ou un groupe phényle mono- ou disubstitué de manière indépendante par un atome d'halogène, un groupe hydroxyle, un groupe cyano, un groupe carboxyle, un groupe -O-alkyle en C₁-C₄, un groupe -O(CH₂C₆H₅), un groupe -COO-alkyle en C₁-C₄, un groupe amino, un groupe diméthylamino, un groupe -NHR¹⁰, un groupe 1-pyrrolidinyle ou un groupe tétrazolyle; soit
(3) un groupe pyridine ou un groupe pyridinyle mono- ou disubstitué de manière indépendante par un atome d'halogène, un groupe méthyle, un groupe hydroxyle, un groupe nitro, un groupe cyano, un groupe carboxyle, un groupe -O-alkyle en C₁-C₄, un groupe -O(CH₂C₆H₅), un groupe -COO-alkyle en C₁-C₄, un groupe amino ou un groupe diméthylamino; soit
(4) un groupe -NHR¹¹ où R¹¹ est tel que défini ci-dessous ou bien R¹¹ représente un groupe 7-indazolyle contenant un groupe R¹⁰ à la position N-1;
R³ représente un atome d'hydrogène, un groupe alkyle en C₁-C₆, un groupe cycloalkyle en C₃-C₆, un groupe phényle ou un groupe phényle mono- ou disubstitué de manière indépendante par un atome d'halogène;
R⁴ représente, de manière indépendante, un groupe alkyle en C₃-C₆, un groupe cycloalkyle en C₃-C₆, un groupe alcényle en C₃-C₆, un groupe phényle, un groupe -(CH₂)ₚCN ou un groupe -(CH₂)ₚCOO-alkyle en C₁-C₄, et R⁵ représente, de manière indépendante, un groupe alkyle en C₃-C₆, un groupe cycloalkyle en C₃-C₆, un groupe alcényle en C₃-C₆, un groupe benzyle, un groupe phényle ou un groupe phényle mono- ou disubstitué de manière indépendante par un groupe alkyle en C₁-C₃, un groupe cyano, un groupe hydroxyle, un groupe diméthylamino, un groupe -O-alkyle en C₁-C₄, un groupe -O(CH₂C₆H₅), un groupe -NH-alkyle en C₁-C₄, un groupe COO-alkyle en C₁-C₄, un groupe -N(alkyle en C₁-C₄)₂, un groupe pyrrolidino, un groupe morpholino ou un atome d'halogène ou bien R⁴ représente un groupe alkyle en C₁-C₂ et R⁵ représente un groupe phényle substitué en position 2 ou en position 4 par un groupe chloro, un groupe méthyle, un groupe méthoxy ou un groupe méthoxycarbonyle;
R⁶ représente un atome d'hydrogène ou un groupe méthyle;
R⁷ représente un atome d'hydrogène, un groupe hydroxyle, un groupe fluoro, un groupe diméthylamino, un groupe -O-alkyle en C₁-C₄ ou un groupe -O(CH₂C₆H₅);
R⁸ représente un groupe -(CH₂)_{b}COOH;
R⁹ représente un groupe méthyle, un groupe chloro, un groupe nitro, un groupe hydroxyle, un groupe méthoxy ou un groupe -NHR¹⁰;
R¹⁰ représente un atome d'hydrogène, un groupe acétyle, un groupe alkyle en C₁-C₄, un groupe -SO₃H, un groupe -SO₂CH₃, un groupe -SO₂CF₃, un groupe -SO₂C₆H₅ ou un groupe alcoxy(en C₁-C₄)-carbonyle;
R¹¹ représente un groupe phényle ou un groupe phényle mono- ou disubstitué de manière indépendante par un atome de fluor, un groupe trifluorométhoxy, un groupe alkyl(en C₁-C₄)thio, un groupe -(CH₂)_{c}COOH, un groupe -(CH₂)_{c}COO-alkyle en C₁-C₄, un groupe -(CH₂)_{c}SCH₃, un groupe -(CH₂)_{c}SOCH₃, un groupe -(CH₂)_{c}SO₂CH₃, un groupe -(CH₂)_{c}CONH₂, un groupe -SCH₂COOH, un groupe -CONH(SO₂CH₃), un groupe -CONH(SO₂CF₃), un groupe -(CH₂)_{c}N-(alkyl en C₁-C₄)₂, un groupe -(CH₂)_{c}NH(SO₂CF₃), un groupe -(CH₂)_{c}N(SO₂CF₃)-alkyle en C₁-C₄, un groupe -(CH₂)_{c}SO₂NHCO-alkyle en C₁-C₄, un groupe -(CH₂)_{c}SO₂N-(alkyl en C₁-C₄)CO-alkyle en C₁-C₄, un groupe -(CH₂)_{c}CONHSO₂-alkyle en C₁-C₄, un groupe -(CH₂)_{c}CON-(alkyl en C₁-C₄)SO₂-alkyle en C₁-C₄, un groupe -(CH₂)_{c}OR¹², un groupe -(CH₂)_{c}NHR¹⁰ ou un groupe phényle mono- substitué par un groupe -(CH₂)_{c}(tétrazolyle), un groupe -(CH₂)_{c}-(carboxamidotétrazolyle) ou un groupe -(CH₂)_{c}(pyrrolidinyle), ou bien R¹¹ est choisi parmi le groupe comprenant un groupe pyridine ou un groupe pyridinyle mono- ou disubstitué de manière indépendante par un atome d'halogène, un groupe méthyle, un groupe hydroxyle, un groupe nitro, un groupe cyano, un groupe carboxyle, un groupe -O-alkyle en C₁-C₄, un groupe amino, un groupe diméthylamino, un groupe NHR¹⁰;
R¹² représente un atome d'hydrogène, un groupe alkyle en C₁-C₆, un groupe cycloalkyle en C₃-C₆, un groupe CH₂C₆H₅, un groupe -CH₂COOH, un groupe -CH₂CONH₂, un groupe CH₂CONH-alkyle en C₁-C₄, un groupe -CH₂CON(alkyle en C₁-C₄)₂ ou le groupe 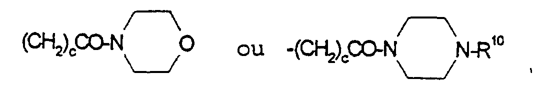
z représente 1 ou 2;
n représente 1 ou 2;
p représente un entier de 1 à 4:
b représente un entier de 0 à 3; et
c représente 0 ou 1.
dans la préparation d'un agent thérapeutique pour contrôler la vidange gastrique chez des patients affectés d'un diabète préliminaire non insulino-dépendant et manifestant une vidange gastrique rapide.

2. Utilisation d'un composé selon la revendication 1, dans laquelle R¹ représente le groupe répondant à la formule (II) dans lequel R⁶ représente un groupe méthyle, R⁷ représente un atome d'hydrogène, un groupe hydroxyle, un groupe méthoxy ou un atome de fluor et n représente 1 ou bien R¹ représente le groupe NR⁴R⁵ dans lequel R⁴ représente un groupe alkyle en C₃-C₆, un groupe cyclohexyle ou un groupe phényle et R⁵ représente un groupe alkyle en C₃-C₆ ou un groupe phényle le cas échéant substitué en position para par un groupe hydroxyle, un groupe diméthylamino, un groupe méthoxy, un atome de fluor, un groupe pyrrolidino ou un groupe morpholino.

3. Utilisation d'un composé selon la revendication 1 ou 2, dans laquelle R¹ représente le groupe NR⁴R⁵ et R⁴ représente un groupe propyle ou un groupe isopropyle et R⁵ représente un groupe phényle ou un groupe phényle substitué en position para par un groupe choisi parmi le groupe comprenant un groupe hydroxyle, un groupe méthoxy, un groupe diméthylamino, un atome de fluor ou un groupe morpholino.

4. Utilisation d'un composé selon l'une quelconque des revendications 1 à 3, dans laquelle R² représente un groupe choisi parmi le groupe comprenant un groupe phényle (le cas échéant substitué par un ou deux groupes qui peuvent être identiques ou différents et qui sont choisis parmi le groupe comprenant un atome de chlore, un atome de fluor, un groupe amino, un groupe hydroxyle ou un groupe carboxyle) ou un groupe NHR¹¹ où R¹¹ représente un groupe phényle (le cas échéant substitué par un atome de fluor, un groupe hydroxyle, un groupe amino, un groupe diméthylamino, un groupe trifluorométhylsulfonylamino, un groupe alcoxy(en C₁-C₄)carbonyle, un groupe carboxyle, un groupe 1H-tétrazol-5-yle, un groupe acétylamino ou un groupe OR¹² où R¹² représente un atome d'hydrogène, un groupe méthyle, un groupe benzyle, un groupe -CH₂CO₂H, un groupe -CH₂CONH₂, un groupe -CH₂CONHCH₃, un groupe -CH₂CON(CH₃)₂, ou encore un groupe 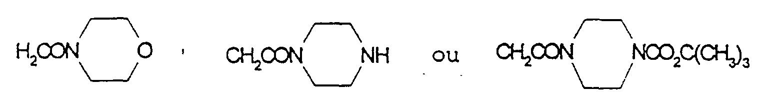 ) ou un groupe 7-indazolyle dans lequel le substituant R¹⁰ de l'atome d'azote en position 1 représente un atome d'hydrogène, ou bien R² représente un groupe indole dans lequel l'atome d'azote est, le cas échéant, substitué par le groupe -CH₂CO₂H et le noyau benzénique est, le cas échéant, substitué par un groupe choisi parmi le groupe comprenant un atome de chlore, un groupe méthyle, un groupe méthoxy, un groupe nitro, un groupe hydroxyle ou un groupe amino.

5. Utilisation d'un composé selon l'une quelconque des revendications 1 à 4, dans laquelle R² représente un groupe indole qui est non substitué sur l'atome d'azote et dans lequel le noyau benzénique est, le cas échéant, substitué par un groupe choisi parmi le groupe comprenant un atome de chlore, un groupe méthyle, un groupe méthoxy, un groupe nitro, un groupe hydroxyle ou un groupe amino.

6. Utilisation d'un composé selon l'une quelconque des revendications 1 à 5, dans laquelle R³ représente un atome d'hydrogène, un groupe méthyle, un groupe cyclohexyle, un groupe 2-fluorophényle ou un groupe phényle.

7. Utilisation d'un composé selon l'une quelconque des revendications 1 à 6, dans laquelle R³ représente un groupe phényle.

8. Utilisation d'un composé selon l'une quelconque des revendications 1 à 7, dans laquelle X représente un atome d'hydrogène.

9. Utilisation d'un composé selon la revendication 1, dans laquelle R¹ représente un groupe NR⁴R⁵ et R⁴ représente un groupe isopropyle et R⁵ représente un groupe p-méthoxyphényle; R² représente un groupe 2-indole non substitué; R³ représente un groupe phényle et X représente un atome d'hydrogène; ainsi que de ses énantiomères.
